# EUROPÄISCHE PATENTSCHRIFT

(11) **EP 0 780 091 B1**
(45) Veröffentlichungstag und Bekanntmachung des Hinweises auf die Patenterteilung: **11.08.1999**
(21) Anmeldenummer: 96119051.9
(22) Anmeldetag: 28.11.1996
(51) Int. Cl.: A61B 17/34

(54) **Medizinische Nadel zum Penetrieren von Körpergewebe**
Medical needle for penetrating body tissues
Aiguille médicale de pénétration des tissus corporels

(30) Priorität: 18.12.1995 DE 19547246
(43) Veröffentlichungstag der Anmeldung: 25.06.1997
(73) Patentinhaber: Riek, Siegfried, Dr. med., D-78667 Villingendorf (DE); Bachmann, Karl-Heinz, D-78667 Villingendorf (DE); Gaiselmann, Thomas, D-78667 Villingendorf (DE)
(72) Erfinder: Riek, Siegfried, Dr. med., D-78667 Villingendorf (DE); Bachmann, Karl-Heinz, D-78667 Villingendorf (DE); Gaiselmann, Thomas, D-78667 Villingendorf (DE)
(74) Vertreter: Patentanwälte Westphal, Mussgnug & Partner

(56) Entgegenhaltungen:
- WO-A-96/01132
- DE-U- 9 112 976

## Beschreibung

Die Erfindung betrifft eine medizinische Nadel gemäß dem Oberbegriff des Anspruchs 1. Eine derartige Nadel ist aus der US-A-4 254 762 bekannt.

Um in der endoskopischen Chirurgie das Risiko zu verringern, beim Einstich Blutgefäße oder innere Organe zu verletzen, wird die sogenannte Veres-Nadel verwendet. Die Veres-Nadel weist eine hohle Außenkanüle auf, die an ihrem distalen Ende zu einer scharfen penetrierenden Spitze schräg angeschliffen ist. In der Kanüle ist axial verschiebbar und abgefedert ein Schutzelement gelagert, welches an seinem vorderen distalen Ende eine geschlossene stumpfe nur leicht gewölbte Schildfläche aufweist. Das Schutzelement ist zum Insufflieren von Gas rohrförmig mit einer hinter der distalen Spitze angeordneten seitlichen Öffnung ausgebildet.

Die Veres-Nadel wird vorzugsweise in der endoskopischen Bauchchirurgie zum Anlegen des Pneumoperitoneums im ersten Einstich eingesetzt. Hierzu wird zunächst eine kleine Hautinzision gemacht, die Veres-Nadel wird dann durch das Unterhautfettgewebe vorgeführt, wobei das Schutzelement durch die Federkraft distal nach vorn gedrückt wird, da der Widerstand im Unterhautfettgewebe kleiner ist als die Federkraft. Die stumpfe Schildfläche des Schutzelementes ragt distal über die scharfe Spitze der Hohlkanüle vor und verdrängt das Unterhautfettgewebe stumpf, so daß keine Gefäßverletzungen im Unterhautfettgewebe durch die scharfe penetrierende Spitze stattfinden. Ist die Faszie erreicht, steigt der Widerstand des Gewebes stark an und wird größer als die Federkraft, die das Schutzelement nach vorn drückt. Die Schildfläche des Schutzelementes weicht hinter die scharfe Spitze zurück und diese kann nun die Faszie penetrieren. Unmittelbar nach der Penetration der Faszie sinkt der Gegendruck des Gewebes wieder ab, so daß die Federkraft das Schutzelement wieder nach vorne schieben kann und die Schildfläche im Bereich des Praeperitoneums die scharfe Spitze wieder schützt. Sobald das Peritoneum erreicht ist, steigt der sich der Spitze entgegenstellende Druck wieder an, so daß das Schutzelement mit der Schildfläche wieder zurückweicht und die scharfe Spitze das Peritoneum eröffnen kann. Sobald die Spitze in den Bauchraum vorgleitet, schnellt das Schutzelement unter der Federkraft wieder nach vorn, da die freie Bauchhöhle keinen Widerstand mehr entgegensetzt. Die stumpfe Schildfläche schützt somit die Gefäße und den Darm in der freien Bauchhöhle vor Verletzungen durch die scharfe Spitze.

Bei der Veres-Nadel wird die Penetration der Faszie und des Peritoneums daran erkannt, daß das Schutzelement nach hinten geschoben wird und nach der Penetration wieder nach vorn schnellt. Auch diese bekannte Veres-Nadel weist ein erhebliches Restrisiko auf. Zum Beispiel werden am Peritoneum perietale adhärente Darmschlingen und im Bereich des Retroperitoneums liegende große Gefäße unter Umständen nicht bemerkt, da die scharfe Spitze unmittelbar nach dem Durchdringen des Peritoneums in diese Darmschlingen oder Gefäße eindringt, ohne daß das Schutzelement nach vorn schnellen kann.

Dieses Restrisiko wird bei einer Nadel, wie sie aus der DE 91 12 976 U1 bekannt ist, verringert. Bei dieser Nadel ist das in dem mit der scharfen Spitze versehenen Kanülenrohr verschiebbare Schutzelement rohrförmig ausgebildet und weist ein offenes distales vorderes Ende auf. In das rohrförmige Schutzelement wird ein optisches System eingesetzt. Das Schutzelement erfüllt somit nicht nur die Schutzfunktion wie bei der herkömmlichen Veres-Nadel, sondern ermöglicht zusätzlich dem Operateur beim Einstich eine optische Beobachtung der durchstochenen Gewebeschichten und damit eine Überprüfung, ob nach dem Durchstechen der Bauchdecke tatsächlich die freie Bauchhöle erreicht ist.

Weiter ist ein Trokar bekannt (DE 40 35 146 C2), dessen penetrierende Spitze als durchsichtiges Fenster ausgebildet ist. Ein in den Trokarschaft eingesetztes optisches System erlaubt die Beobachtung sowohl des unmittelbar vor der Spitze liegenden Gewebes als auch des seitlich an der Spitze anliegenden Gewebes beim Eindringen der Trokarspitze. Der Operateur kann daher das Gewebe unter Sicht penetrieren. Dieser Trokar hat jedoch einen relativ großen Außendurchmesser. Bei dünnen Nadeln von nur 2 bis 3 mm Außendurchmessern ist eine durchsichtige Spitze mit den für die Penetration notwendigen mechanischen Eigenschaften schwierig herzustellen.

Aus der EP 0 684 016 A2 und der EP 0 642 764 A1 ist ein Trokar bekannt, bei welchem die distale Spitze abgerundet und durchsichtig ist. Das Eindringen der Spitze in das Gewebe kann über eine in den Trokar eingesetzte Optik beobachtet werden. Da sich die abgerundete Spitze des Trokars nicht zum Penetrieren des Gewebes eignet, sind in der Spitze scharfe Schneiden angeordnet, die distal nach vorn über die abgerundete durchsichtige Trokarspitze hinausbewegt werden können, um das zu penetrierende Gewebe zu durchtrennen. Hierbei ergibt sich der Nachteil, daß bei dem eigentlichen Penetrationsvorgang mittels der distal ausgefahrenen Schneiden eine Beobachtung nicht möglich ist.

Bei der aus der US-A-4 254 762 bekannten Nadel ist das Schutzelement rohrförmig ausgebildet und weist an seinem distalen vorderen Ende ein durchsichtiges Dichtungsmaterial, bzw. eine Linse, auf.

Der Erfindung liegt die Aufgabe zugrunde, eine medizinische Nadel zur Verfügung zu stellen, die eine scharfe penetrierende Spitze aufweist und bei der das Risiko der Verletzung von Gefäßen und Organen durch die scharfe penetrierende Spitze möglichst gering ist, wodurch eine bessere Beobachtung des Penetrationsvorgangs gewährleistet ist.

Diese Aufgabe wird bei einer Nadel der eingangs genannten Gattung erfindungsgemäß gelöst, durch die Merkmale des kennzeichnenden Teiles des Anspruchs 1.

Vorteilhafte Ausführungen der Erfindungen sind in den Unteransprüchen angegeben.

Die chirurgische Nadel weist eine scharfe penetrierende Spitze mit einem abgefedert verschiebbaren Schutzelement auf, dessen distale stumpfe Schildfläche abgerundet und optisch durchsichtig ausgebildet ist. In dieses Schutzelement ist ein optisches System eingesetzt, mit welchem die Schildfläche von der Innenseite beobachtet werden kann.

Das optische System ist in dem Schutzelement so angeordnet, daß das distale Objektiv des Systems axial gegenüber der durchsichtigen Schildfläche beabstandet ist, so daß sich eine gute optische Abbildung der an der Schildfläche anliegenden Gewebepartien ergibt. Das optische System kann fest in das Schutzelement eingesetzt sein oder auswechselbar in dem Schutzelement angeordnet werden. Eine auswechselbare Anordnung hat den Vorteil, daß die Nadel als Einmal-Instrument ausgebildet sein kann, während die aufwendigere Optik mehrfach verwendbar ist. Auch bei einem Mehrfach-Instrument kann die Austauschbarkeit des optischen Systems von Vorteil sein, da das optische System bei der Sterilisation der Nadel herausgenommen werden kann und nicht den hohen Sterilisationstemperaturen ausgesetzt werden muß.

Die stumpfe Schildfläche schirmt in der distal vorgeschobenen Stellung des Schutzelementes die scharfe Spitze möglichst gut ab, da die scharfe penetrierende Spitze an der zylindrischen Mantelfläche des Schutzelements anliegt, so daß die Spitze trotz der Abrundung der Schildfläche nicht frei vorragt.

Die Form der stumpfen Schildfläche des Schutzelements kann nach den jeweiligen Anforderungen gestaltet werden. Eine flache wenig nach vorn gewölbte Schildfläche bedeutet eine großflächigere Abstützung an dem Gewebe, so daß ein größerer der Federkraft entgegenwirkender Gewebedruck auf das Schutzelement ausgeübt wird.

Der bei der herkömmlichen Veres-Nadel ausgenützte Effekt des sich ändernden Gewebswiderstands ist in dieser Ausführung ebenfalls verwendbar.

Eine stärkere Auswölbung der Schildfläche nach vorn mit einem kleineren Krümmungsradius kann unter anderen Gesichtspunkten von Vorteil sein. Der kleinere Krümmungsradius und die stärkere Wölbung der Schildfläche bedeuten einen geringeren Gegendruck des Gewebes auf die Schildfläche und das Schutzelement bei dem Penetrationsvorgang. Der geringere Druck der Schildfläche auf das Gewebe hat zur Folge, daß das Gewebe weniger anämisch wird, d.h. daß das Blut weniger aus dem Gewebe gedrückt wird, wodurch das Gewebe ein blaßhelles Aussehen erhalten würde. Außerdem kann das Schutzelement mit der stumpfen vorgewölbten Schildfläche auch bewußt nach vorn gedrückt und in der vorderen Stellung gehalten werden, um sich mit der Spitze in dem Gewebe unter Sicht vorwärts zu tasten und die scharfe penetrierende Spitze unter Freigabe des Schutzelements nur dann zum Einsatz kommen zu lassen, wenn eine gefahrlose Penetration gewährleistet ist.

Beim Einstich und bei dem Vorschieben der Nadel erscheint zunächst das Unterhautfettgewebe gelblich. Ist die Faszie erreicht, so erscheint diese weiß. Nach der Penetration der Faszie sieht man die Muskulatur rot. Eventuell vor der Spitze der Nadel liegende Gefäße können erkannt und umgangen werden. Ist das praeperitoneale Fettgewebe erreicht, so kann gegebenenfalls das Schutzelement mit der stumpfen Schildfläche bewußt nach vorn gedrückt werden, so daß die stumpfe Schildfläche unabhängig von der Federkraft über die scharfe Spitze hinausragend festgehalten wird. Es kann mit der stumpfen Schildfläche das Fettgewebe verdrängt werden, bis die Schildfläche an dem Peritoneum perietale anliegt. Dieses kann mit der stumpfen Schildfläche ohne Penetrierung eingedrückt und gespannt werden (Tenting-Effekt). Dadurch wird das Peritoneum semitransparent und an dem Peritoneum adhärente Darmschlingen oder Gefäße können erkannt werden.

Ist auf diese Weise sichergestellt, daß keine Adhäsionen unter dem Peritoneum vorhanden sind, wird das Schutzelement freigegeben, so daß es hinter die scharfe penetrierende Spitze zurückweicht. Die scharfe Spitze penetriert dann risikolos das Peritoneum.

Im folgenden wird die Erfindung an Hand eines in der Zeichnung dargestellten Ausführungsbeispiels näher erläutert. Es zeigen:
- Figur 1: eine Nadel mit dem Schutzelement in der hinteren Stellung,
- Figur 2: die Nadel mit dem Schutzelement in der vorderen Stellung,
- Figur 3: die distale Spitze der Nadel in der hinteren Stellung des Schutzelementes in einer vergrößerten Seitenansicht,
- Figur 4: eine um 90° gedrehte Darstellung der Figur 3,
- Figur 5: die distale Spitze der Nadel mit dem Schutzelement in der vorderen Stellung in einer Figur 3 entsprechenden Darstellung und
- Figur 6: eine um 90° gedrehte Darstellung der Figur 5.

In der Zeichnung ist die medizinische Nadel in einer Ausführung dargestellt, wie sie in der endoskopischen Chirurgie für den ersten Einstich zum Anlegen eines Pneumoperitoneums verwendet wird.

Die Nadel weist ein äußeres hohles Kanülenrohr 10 aus Stahl auf, welches beispielsweise einen Außendurchmesser von 1,5 bis 3 mm aufweist. Das distale vordere Ende des Kanülenrohres 10 ist schräg angeschliffen, so daß eine scharfe Spitze 12 gebildet wird, die zum Penetrieren des Gewebes dient. Das Kanülenrohr 10 weist je nach Verwendungszweck eine Länge von etwa 80 mm bis 150 mm auf.

In dem Kanülenrohr 10 ist axial verschiebbar ein Schutzelement 14 angeordnet. Das Schutzelement 14 ist ein koaxial in dem Kanülenrohr 10 geführtes Rohr, welches an seinem distalen vorderen Ende geschlossen ist. Das geschlossene distale Ende des Schutzelementes 14 bildet eine vordere stumpfe Schildfläche 16 des Schutzelementes 14, die sich von der Mantelfläche des Schutzelementes 14 nach vorn ballig verjüngt und am distalen Ende eine kugelkalottenförmige Stirnfläche bildet.

Das mit der Schildfläche 16 versehene distale Ende des Schutzelementes 14 ist optisch durchsichtig und besteht aus Glas oder einem durchsichtigen Kunststoff. Hierzu kann entweder das gesamte rohrförmige Schutzelement 14 aus Glas oder Kunststoff bestehen oder es kann das vordere aus Glas oder Kunststoff bestehende Ende an ein metallisches Rohrteil angesetzt werden. Die axiale Länge des durchsichtigen distalen Endes des Schutzelementes 14 beträgt vorzugsweise mindestens 5 mm. Das mit der Schildfläche 16 versehene durchsichtige Ende des Schutzelementes 14 ist ebenfalls hohl, wobei der innere Hohlraum 17 kuppelförmig ausgebildet ist, d. h. nach vorn zu spitz oder abgerundet zuläuft.

In das rohrförmige Schutzelement 14 wird von dessen proximalem Ende her ein stabförmiges optisches System 18, z. B. ein Stablinsensystem eingeführt. Das optische System 18 wird so weit in das Schutzelement 14 eingeschoben, daß das distale Objektiv 20 des Systems 18 sich in einem axialen Abstand hinter dem distalen Ende der Schildfläche 16 und des Innenhohlraums 17 der Schildfläche 16 befindet. In dieser Position rastet das optische System 18 in dem Schutzelement 14 ein, so daß es axial fest in dem Schutzelement 14 gehalten ist und sich bei der axialen Verschiebung des Schutzelementes 14 mit diesem bewegt.

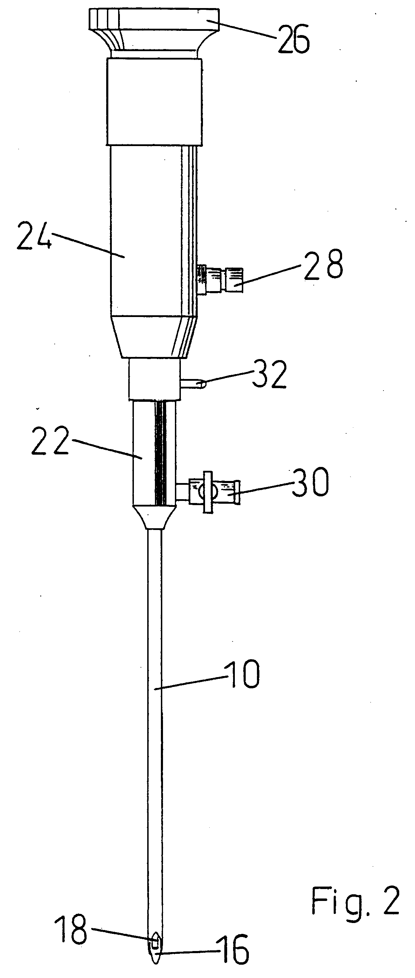

Das Schutzelement 14 ist in dem äußeren Kanülenrohr 10 durch eine am proximalen Ende angeordnete Druckfeder axial abgefedert. Die Druckfeder schiebt das Schutzelement 14 mit einer vorgegebenen Federkraft axial nach vorn in die in den Figuren 2, 5 und 6 gezeigte Stellung, in welcher die Schildfläche 16 des Schutzelementes 14 über die scharfe Spitze 12 des Kanülenrohres 10 distal hinausragt.

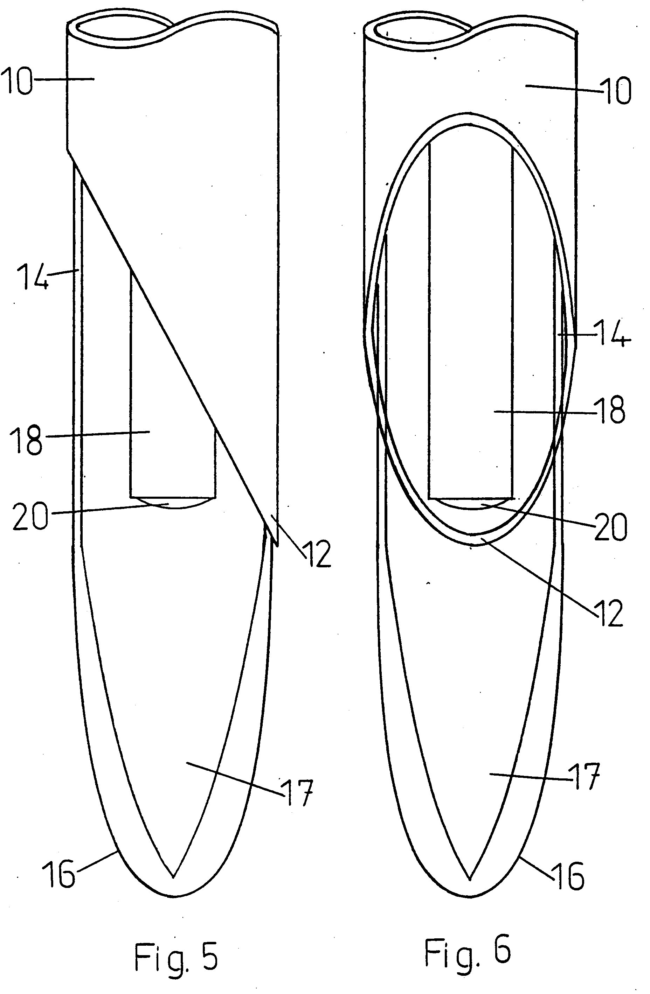

Der distale Vorschubweg ist dabei so begrenzt, daß in der distalen Endstellung, die in den Figuren 5 und 6 gezeigt ist, die Spitze 12 in dem Bereich liegt, in welchem die gekrümmte Schildfläche 16 in die zylindrische Außenmantelfläche des Schutzelementes 14 übergeht. Dadurch liegt die scharfe Spitze 12 dicht am Mantel des Schutzelementes 14 an und wird durch dieses abgeschirmt.

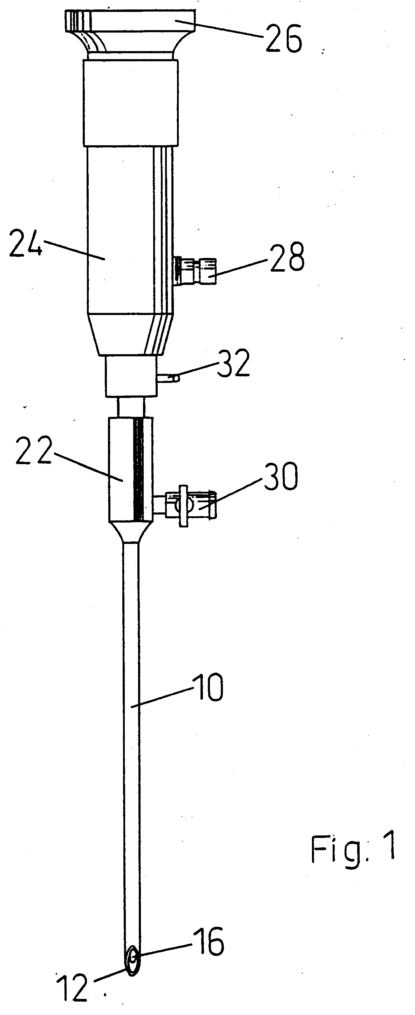

Das Schutzelement 14 ist gegen die Kraft der Druckfeder axial nach hinten in eine hintere Endstellung verschiebbar, in welcher die Schildfläche 16 hinter die scharfe Spitze 12 des Kanülenrohres 10 zurückweicht, wie dies in den Figuren 1, 3 und 4 gezeigt ist. In dieser hinteren Endstellung des Schutzelementes 14 ragt die scharfe Spitze 12 frei nach vorn und kann unbehindert durch die Schildfläche 16 das Gewebe penetrieren.

Das Kanülenrohr 10 weist an seinem hinteren proximalen Ende einen Griffansatz 22 auf. Das Schutzelement 14 ist mit seinem hinteren proximalen Ende auf ein Kopfteil 24 aufsetzbar. Das Kopfteil 24 weist an seinem proximalen Ende ein Okular 26 auf, über welches die Schildfläche 16 mittels des optischen Systems 18 beobachtet werden kann. Weiter weist das Kopfteil 24 einen seitlichen Anschluß 28 auf, an welchen eine Lichtquelle und gegebenenfalls eine Kamera oder ein Bildwandler an das optische System 18 angeschlossen werden können.

An dem Griffansatz 22 ist ein Ventilhahn 30 vorgesehen, über welchen CO₂-Gas zugeführt und durch das Kanülenrohr 10 oder durch einen in dem Kanülenrohr 10 angeordneten Kanal zu der Spitze geleitet werden kann, um in die Bauchhöhle insuffliert zu werden.

Beim Einstich der Nadel wird das Kanülenrohr 10 mit dem Griffansatz 22 geführt. Das Kopfteil 24 verschiebt sich mit dem Schutzelement 14 gegenüber dem Kanülenrohr 10. Vorn an dem Kopfteil 24 ist ein Hebel 32 angebracht, mit dem das optische System 18 im Schutzelement 14 arretiert werden kann.

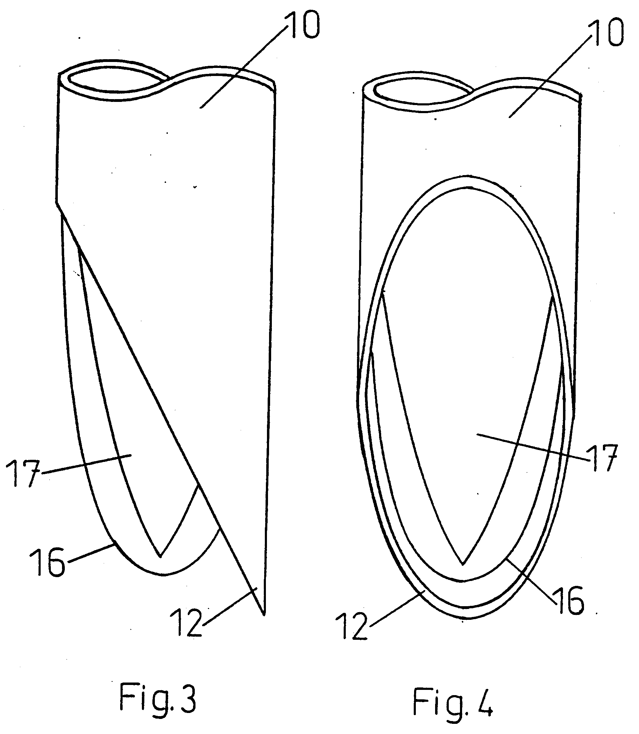

Beim Penetrieren des Gewebes schiebt sich das Schutzelement 14 in die in den Figuren 5 und 6 gezeigte vordere Stellung oder wird in die in den Figuren 3 und 4 gezeigte hintere Stellung zurückgedrückt, je nachdem ob der durch den Widerstand des penetrierten Gewebes auf die Schildfläche 16 ausgeübte Druck kleiner oder größer als die auf das Schutzelement 14 wirkende Federkraft ist. Das optische System 18 sitzt axial arretiert in dem Schutzelement 14 und bewegt sich bei der axialen Verschiebung des Schutzelementes 14 mit diesem. Das Objektiv 20 befindet sich somit immer in dem vorgegebenen axialen Abstand von dem distalen Ende der Schildfläche 16, so daß eine optimale optische Beobachtung des an der Schildfläche 16 anliegenden Gewebes über den Innenhohlraum 17 der Schildfläche 16 möglich ist.

Bei dieser Ausführungsform wird beim Einstich der Nadel das Kanülenrohr 10 an dem Griffansatz 22 geführt. Da sich das Kopfteil 24 mit dem Schutzelement 14 gegenüber dem Kanülenrohr verschiebt, kann das relativ schwere Kopfteil 24 beim Einstich der Nadel nicht vom Operateur gehalten werden und erzeugt ein Kippmoment auf die Nadel, das die Führung der Nadel beeinträchtigen kann.

In einer anderen Ausführungsform ist deshalb das optische System 18 axial verschiebbar in dem Schutzelement 14 aufgenommen. Dadurch kann das Kanülenrohr 10 an dem Kopfteil 24 befestigt werden. Beim Einstich der Nadel kann das Kanülenrohr 10 und das mit diesem verbundene Kopfteil 24 von der Hand des Operateurs geführt werden, so daß das Gewicht des Kopfteils 24 nicht störend wirkt. Das Schutzelement 14 verschiebt sich je nach dem Gegendruck des Gewebes axial sowohl in Bezug auf das Kanülenrohr 10 als auch in Bezug auf das optische System 18. Dadurch verändert sich der axiale Abstand zwischen der Schildfläche 16 und dem Objektiv 20 des optischen Systems 18. Durch eine geeignete Ausbildung des optischen Systems und insbesondere des Objektives 20 kann sichergestellt werden, daß diese Abstandsänderung die Beobachtung der Schildfläche 16 über das optische System 18 nicht wesentlich beeinträchtigt.

### Bezugszeichenliste

- 10: Kanülenrohr
- 12: Spitze
- 14: Schutzelement
- 16: Schildfläche
- 17: Innenhohlraum
- 18: optisches System
- 20: Objektiv
- 22: Griffansatz
- 24: Kopfteil
- 26: Okular
- 28: Anschluß
- 30: Ventilhahn
- 32: Hebel

## Patentansprüche

1. Medizinische Nadel
mit einem Kanülenrohr (10), dessen distales Ende zu einer scharfen Spitze (12) abgeschrägt ist,
mit einem koaxial in dem Kanülenrohr (10) angeordneten rohrförmigen Schutzelement (14), welches aus einer distal vorderen Stellung gegen eine Federkraft in eine hintere Stellung axial bewegbar ist und welches an seinem distalen Ende durch eine durchsichtige stumpfe Schildfläche (16) abgeschlossen ist, die in der vorderen Stellung distal über die Spitze (12) hinausragt und in der hinteren Stellung hinter die Spitze (12) zurückweicht, und mit einem in das Schutzelement (14) einsetzbaren optischen System (18), dessen distales Objektiv (20) zur Beobachtung der Schildfläche (16) von innen dient,
dadurch **gekennzeichnet**,
daß die Schildfläche (16) an ihrem distalen Ende nach vorne ausgewölbt ist,
daß das Schutzelement (14) bis in den ausgewölbten Bereich der Schildfläche (16) hohl ist, so daß innerhalb der Schildfläche (16) ein **kuppelförmiger** Innenhohlraum (17) gebildet ist, daß das Objektiv (20) des eingesetzten optischen Systems im Inneren des Schutzelements (14) axial von der Schildfläche (16) beabstandet ist, und daß in der distalen Endstellung des Schutzelements (14) die Spitze (12) des Kanülenrohres (10) in dem Bereich liegt, in welchem die gewölbte Schildfläche (16) in die zylindrische Außenmantelfläche des Schutzelements (14) übergeht.

2. Nadel nach Anspruch 1, dadurch gekennzeichnet, daß das optische System (18) auswechselbar in das Schutzelement (14) einsetzbar ist.

3. Nadel nach Anspruch 1, dadurch gekennzeichnet, daß das distale Objektiv (20) des eingesetzten optischen Systems (18) etwa in der Basisfläche des kuppelförmigen Innenhohlraumes (17) angeordnet ist.

4. Nadel nach Anspruch 2, dadurch gekennzeichnet, daß das optische System (18) in dem Schutzelement (14) axial arretierbar und zusammen mit dem Schutzelement (14) bewegbar ist.

5. Nadel nach Anspruch 2, dadurch gekennzeichnet, daß das optische System (18) in dem Schutzelement (14) axial verschiebbar angeordnet und zusammen mit dem Kanülenrohr (10) an einem Kopfteil (24) befestigbar ist.

## Claims

1. A medical needle,
having a cannula tube (10), the distal end of which is chamfered to a sharp point,
having a tubular protective element (14) disposed coaxially in the cannula tube (10), which can be axially moved out of a distally front position against a spring force into a rear position and which at its distal end is closed by a transparent, blunt shield face (16), which in the front position juts out distally over the point (12) and in the rear position retreats behind the point (12), and having an optical system (18) insertable into the protective element (14), the distal lens (20) of which is used to observe the shield face (16) from inside,
**characterised in that** the shield face (16) is forwardly curved at its distal end,
**in that** the protective element (14) is hollow right up to the outwardly curved region of the shield face (16), so that inside the shield face (16) a dome-shaped inner cavity is formed,
**in that** the lens (20) of the inserted optical system inside the protective element (14) is axially spaced from the shield face (16),
**and in that** in the distal end position of the protective element (14) the point (12) of the cannula tube (10) lies in the region in which the curved shield face (16) merges into the cylindrical outer surface of the protective element (14).

2. A needle according to Claim 1,
**characterised in that** the optical system (18) can be exchangeably inserted into the protective element (14).

3. A needle according to Claim 1,
**characterised in that** the distal lens (20) of the inserted optical system (18) is disposed for instance in the base of the dome-shaped inner cavity (17).

4. A needle according to Claim 2,
**characterised in that** the optical system (8) can be axially fixed in the protective element (14) and can move together with the protective element (14).

5. A needle according to Claim 2,
**characterised in that** the optical system (8) is axially displaceably disposed in the protective element (14) and can be fixed together with the cannula tube (10) on a head part (24).

## Revendications

1. Aiguille médicale, équipée d'un tube de canule (10) dont l'extrémité distale est biseautée pour former une pointe (12) tranchante, avec un élément protecteur (14) en forme de tube, disposé coaxialement dans le tube de canule (10) et déplaçable axialement depuis une position avant distale, à l'encontre d'une force élastique, en une position arrière et qui est fermé à son extrémité distale au moyen d'une face formant bouclier (16) sans tranchant, transparente qui, dans la position avant fait saillie de façon distale par la pointe (12) et qui, dans la position arrière, se rétracte derrière cette pointe (12), et avec un système optique (19) pouvant être inséré dans l'élément protecteur (14) et dont l'objectif distal (20) sert à l'observation depuis l'intérieur de la face formant bouclier (16),
caractérisée en ce que
• la face formant bouclier (16) est bombée vers l'avant à son extrémité distale,
• l'élément protecteur (14) est creux jusque dans la zone bombée et creusée de la face formant bouclier (16), de manière que, à l'intérieur de la face formant bouclier (16), soit constitué un espace creux intérieur (17) en forme de coupole, en ce que l'objectif (20) du système optique inséré, placé à l'intérieur de l'élément protecteur (14), est espacé axialement de la surface formant bouclier (16), et,
• dans la position finale de l'élément protecteur (14), la pointe (12) du tube de canule (10) est située dans la zone dans laquelle la surface formant bouclier (16) incurvée se transforme en la surface d'enveloppe extérieure cylindrique de l'élément protecteur (14).

2. Aiguille selon la revendication 1,
caractérisée en ce que
le système optique (18) est insérable de façon remplaçable dans l'élément protecteur (14).

3. Aiguille selon la revendication 1,
caractérisée en ce que
l'objectif distal (20) du système optique (18) inséré est disposé à peu près dans la face de base de l'espace creux intérieur (17) en forme de coupole.

4. Aiguille selon la revendication 2,
caractérisée en ce que
le système optique (18) est susceptible d'être bloqué axialement dans l'élément protecteur (14) et est déplaçable conjointement avec cet élément protecteur (14).

5. Aiguille selon la revendication 2,
caractérisée en ce que
le système optique (18) est disposé de façon déplaçable axialement dans l'élément protecteur (14) et est susceptible d'être fixé sur une partie de tête (24), conjointement avec le tube de canule (10).
